# EUROPEAN PATENT SPECIFICATION

(11) **EP 3 124 073 B1**
(45) Date of publication and mention of the grant of the patent: **30.01.2019**
(21) Application number: 15770175.6
(22) Date of filing: 03.03.2015
(51) Int. Cl.: A61M 39/26

(54) **MEDICAL CONNECTOR**
MEDIZINISCHER VERBINDER
RACCORD MÉDICAL

(30) Priority: 28.03.2014 JP 2014069206
(43) Date of publication of application: 01.02.2017
(73) Proprietor: Terumo Kabushiki Kaisha, Tokyo 151-0072 (JP)
(72) Inventor: UEDA, Yasuhiro, Nakakoma-gun Yamanashi 409-3853 (JP)
(74) Representative: Casalonga
(86) International application number: PCT/JP2015/001123
(87) International publication number: WO 2015/145998

(56) References cited:
- DE-U1-202014 103 247
- JP-A- 2000 503 096
- JP-A- 2008 540 045
- JP-A- 2012 005 605
- JP-A- 2013 059 390
- JP-U- 3 166 779
- US-A- 5 242 393
- US-A1- 2006 293 629
- US-A1- 2011 028 914
- US-A1- 2013 060 205

## Description

### Technical Field

The present invention relates to a medical connector including a housing having a male connector connection section and a medical instrument connection section and a valve element having a head section that closes the male connector connection section and a body section that connects to the head section. In particular, the present invention is to suppress or prevent occurrence of drawing-in of fluid at the medical instrument connection section when removing the male connector from the male connector connection section and suppress or prevent occurrence of contamination due to adhesion of fluid at the body section of the valve element.The present invention relates to a medical connector according to the preamble of claim 1, such as it is, e.g., known from US 2006/293629 or US 2013/060205.

### Background Art

Conventionally, as a medical connector which is used in, for example, various medical apparatuses, infusion containers, and liquid feeding instruments and which connects tube bodies of a medical instrument, for example, a medical connector as described in Patent Literature 1 is known. In Patent Literature 1, a valve element having a head section and a body section is housed in a housing of the medical connector. The body section of the valve element has a hollow section. An air passage that connects the hollow section of the body section with the outside of the housing is formed in the housing.

When connecting the male connector to the male connector connection section, the head section of the valve element is pushed in by the male connector while the head section contracts the body section and a fluid flow passage in the male connector connects to a fluid flow passage in a medical instrument connection section through a fluid connection passage formed between the head section and the body section of the valve element and the housing.

### Citation List

### Patent Literature

Patent Literature 1: JP 2013-500128 A

### Summary of Invention

### Technical Problem

In such a medical connector, it is possible to expand and restore the body section of the valve element when removing the male connector while releasing the connection of the male connector, so that it is possible to suppress or prevent fluid from being drawn into the housing at a medical instrument connection section when removing the male connector. If the fluid is drawn into the inside of the housing from the medical instrument connection section when removing the male connector, for example, when the medical connector is used by being connected to a catheter indwelled in a blood vessel, there is a risk that a problem as described below occurs.

Specifically, when a male connector is connected to a male connector connection section of the medical connector and a blood anticoagulant is injected through the male connector and thereafter the male connector is removed, blood is drawn into the catheter by drawing-in of fluid from the medical instrument connection section to which the catheter is connected, so that the blood clots in the catheter and the catheter is clogged to become unusable. The medical connector as described in Patent Literature 1 can avoid the occurrence of such a problem.

However, in the medical connector as described in Patent Literature 1, when the male connector is connected to the male connector connection section, a fluid from the male connector flows along the body section of the valve element, so that the fluid attaches to and remains on the body section of the valve element and there is a risk that contamination will occur on the body section of the valve element.

The present invention is made in view of the situation described above and an object of the present invention is to provide a medical connector that can suppress or prevent occurrence of drawing-in of fluid at the medical instrument connection section when removing the male connector from the male connector connection section and suppress or prevent occurrence of contamination due to adhesion of fluid at the body section of the valve element.

### Solution to Problem

A medical connector according to the present invention includes : a housing including a male connector connection section and a medical instrument connection section; and a valve element including a head section that closes the male connector connection section and a body section that connects to the head section, wherein the body section of the valve element is arranged in a receiving recess section formed in the housing, and by connecting a male connector to the male connector connection section, the head section of the valve element is pushed in by the male connector while the head section contracts the body section, a fluid flow passage in the male connector is connected to a fluid flow passage in the medical instrument connection section through a fluid connection passage formed outside the receiving recess section, and the body section is covered by the receiving recess section.

Here, in the medical connector according to the present invention, it is preferable that on an outer circumferential surface of the valve element, a notch is formed which connects the fluid flow passage in the male connector to the fluid flow passage in the medical instrument connection section through the fluid connection passage by tilting the head section when the male connector is connected, and a rotation preventing means that prevents rotation of the body section in a circumferential direction is provided between the body section of the valve element and the receiving recess section of the housing.

Furthermore, in the medical connector according to the present invention, it is preferable that the body section of the valve element has a hollow section that opens at an end section opposite to the head section, and the housing has an air passage that connects outside of the housing with the hollow section of the valve element.

Furthermore, in the medical connector according to the present invention, it is preferable that the body section of the valve element has a bellows-shaped circumferential wall that forms the hollow section, and on a bottom surface of the receiving recess section of the housing, a support protrusion is formed which comes into contact with an end surface of the hollow section facing the head section when the bellows-shaped circumferential wall is folded when the male connector is connected and suppresses further deformation of the bellows-shaped circumferential wall.

Furthermore, in the medical connector according to the present invention, it is preferable that a seal protrusion that is liquid-tightly and slidably attached to the receiving recess section of the housing is provided around an outer circumferential surface of the body section of the valve element at an end section of the bellows-shaped circumferential wall facing the head section.

### Advantageous Effects of Invention

According to the present invention, when the male connector is connected to the male connector connection section, the male connector contracts the body section of the valve element when the male connector pushes the head section of the valve element. On the other hand, when the male connector is removed along with disconnection of the male connector from the male connector connection section, it is possible to expand and restore the body section of the valve element. Therefore, it is possible to suppress or prevent the increase of volume of the fluid flow passage in the housing, which has been concerned to occur when the male connector is removed, by expansion of the body section of the valve element, so that it is possible to suppress or prevent occurrence of drawing-in of the fluid at the medical instrument connection section when the male connector is removed.

Further, according to the present invention, when the male connector is connected to the male connector connection section, the body section of the valve element is covered by the receiving recess section of the housing and the fluid flow passage in the male connector connects to the fluid flow passage in the medical instrument connection section through a fluid connection passage formed outside the receiving recess section, so that it is possible to suppress or prevent fluid flowing in the housing from coming into contact with the body section of the valve element. Therefore, it is possible to suppress or prevent occurrence of contamination due to adhesion of fluid at the body section of the valve element.

Therefore, according to the present invention, it is possible to provide a medical connector that can suppress or prevent occurrence of drawing-in of fluid at the medical instrument connection section when removing the male connector from the male connector connection section and suppress or prevent occurrence of contamination due to adhesion of fluid at the body section of the valve element.

### Brief Description of Drawings

Fig. 1 is a vertical cross-sectional view of a medical connector according to a first embodiment of the present invention.
Fig. 2 is a perspective view of the medical connector in Fig. 1.
Fig. 3 is a perspective view of a valve element of the medical connector in Fig. 1.
Fig. 4 is a perspective view of the valve element in Fig. 3 as seen from another angle.
Fig. 5 is a perspective view of a holder of the medical connector in Fig. 1.
Fig. 6 is a vertical perspective view of the medical connector in Fig. 1 and illustrates a state in which a male connector is connected.

### Description of Embodiment

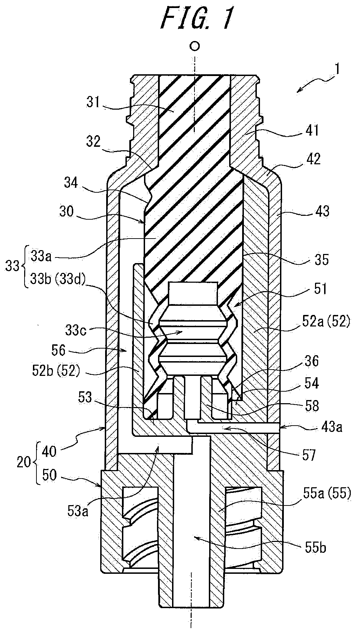

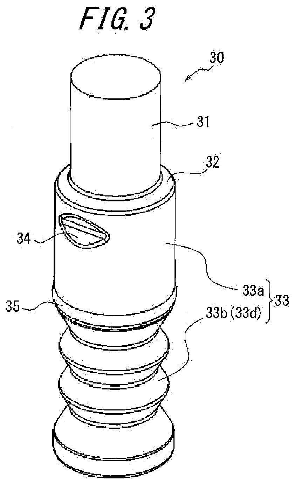

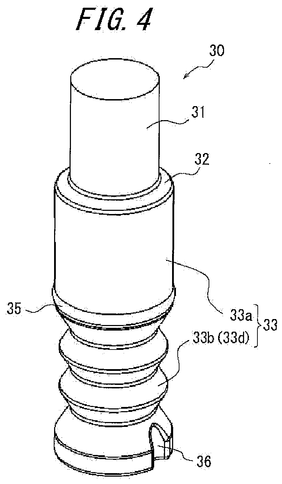

Hereinafter, a medical connector according to an embodiment of the present invention will be described with reference to Figs. 1 to 6.

In the present description, the vertical direction means a direction along an axis line of a medical connector, an upper side means a side on which a male connector connection section is arranged (in other words, an upper side in Fig. 1), and a lower side means a side on which a medical instrument connection section is arranged (in other words, a lower side in Fig. 1) .

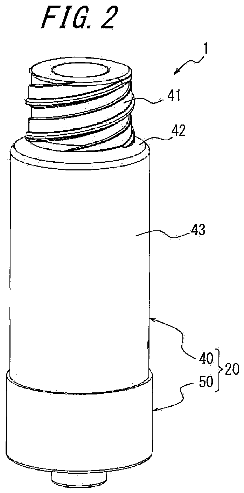

As illustrated in Figs. 1 and 2, a medical connector 1 includes a housing 20 and a valve element 30. In the present embodiment, the housing 20 includes a cap 40 and a holder 50 and is formed by fixing the cap 40 to the holder 50.

The cap 40 has a cylindrical male connector connection section 41 at its upper end. On an outer circumference surface of the male connector connection section 41, in this example, a triple threaded screw to connect a lure lock type male connector is formed. The triple threaded screw can be omitted. A cylindrical outer circumferential wall section 43 is vertically formed at a lower end of the male connector connection section 41 through a taper wall section 42 whose diameter increases in taper form.

As illustrated in Figs. 1, 3, and 4, the valve element 30 has a column-shaped head section 31 that closes the male connector connection section 41. A column-shaped body section 33 is provided in a coupled manner to the lower end of the head section 31 through a shoulder section 32 whose diameter increases in taper form in the same angle as that of the taper wall section 42 of the cap 40.

An upper part 33a of the body section 33 is formed into a solid shape and a lower part 33b of the body section 33 is formed into a hollow shape. In other words, the body section 33 has a hollow section 33c that opens at an end section opposite to the head section 31. A notch 34 having a V-shaped cross-sectional shape is formed near the upper end of the outer circumferential surface of the upper part 33a of the body section 33. A seal protrusion 35 that is liquid-tightly and slidably attached to a receiving recess section 51 described later is provided around the lower end of the outer circumferential surface of the upper part 33a of the body section 33.

The lower part 33b of the body section 33 has a bellows-shaped circumferential wall 33d that forms the hollow section 33c. A recess section 36 that prevents circumferential rotation of the body section 33 by engaging with a protrusion 54 formed in the receiving recess section 51 described later is formed at the lower end of the bellows-shaped circumferential wall 33d. In the present example, the recess section 36 is arranged opposite to the notch 34 with an axis line O in between.

As illustrated in Figs. 1, 2, and 5, the holder 50 has a cylindrical inner circumferential wall section 52 which forms the inner circumferential surface of the ring-shaped receiving recess section 51 in which the body section 33 of the valve element 30 is arranged. Regarding the inner circumferential wall section 52, a first half circumferential part 52a around the axis line O is inserted inside the outer circumferential wall section 43 of the cap 40, the outer circumferential surface of the half circumferential part 52a is in liquid-tightly contact with the inner circumferential surface of the outer circumferential wall section 43 of the cap 40, and the upper surface of the half circumferential part 52a is in liquid-tightly contact with the lower surface of the taper wall section 42 of the cap 40. The remaining half circumferential part around the axis line O of the inner circumferential wall section 52, that is, a second half circumferential part 52b, is smaller than the first half circumferential part 52a in diameter. The size of the inner diameter of the second half circumferential part 52b is the same as that of the first half circumferential part 52a. The upper end of the second half circumferential part 52b is located lower than the upper end of the first half circumferential part 52a.

The lower ends of the first and the second half circumferential parts 52a and 52b are closed by a recess section bottom wall 53 that forms the bottom surface of the receiving recess section 51. The protrusion 54 that engages with the recess section 36 of the valve element 30 described above is formed at the lower end of the inner circumferential surface of the first half circumferential part 52a. The protrusion 54 is located opposite to the center of the second half circumferential part 52b in the circumferential direction with the axis line O in between. The arrangement of the protrusion 54 and the recess section 36 may be appropriately changed in order to prevent the valve element 30 from rotating in the circumferential direction. However, even in such a case, as in the present example, when the body section 33 of the valve element 30 is arranged in the receiving recess section 51, an arrangement is preferable in which the notch 34 of the body section 33 can be positioned at the center of the second half circumferential part 52b in the circumferential direction.

A medical instrument connection section 55 is provided under the recess section bottom wall 53. In the present example, the medical instrument connection section 55 is formed as a lure lock type male connector in which a cylindrical body 55a extends along the axis line O. In the present example, the medical connector 1 is formed as a mixed injection plug having one male connector as the medical instrument connection section 55. However, the medical connector 1 can be formed as, for example, a T-type mixed injection port having a further connection section and a three-way cock instead of the above.

A half-disk-shaped notch 53a positioned below the second half circumferential part 52b of the inner circumferential wall section 52 is formed below the recess section bottom wall 53 and a fluid connection passage 56 formed outside the receiving recess section 51 and a fluid flow passage 55b in the medical instrument connection section 55 are connected to each other by the notch 53a.

A holder side air passage 57 that connects the hollow section 33c of the valve element 30 with a through hole 43a formed in the outer circumferential wall section 43 of the cap 40 is formed in the recess section bottom wall 53. In other words, the housing 20 has the through hole 43a and the holder side air passage 57 as an air passage that connects the outside of the housing 20 and the hollow section 33c of the valve element 30. Further, a cylindrical support protrusion 58 having the axis line O as its center is disposed upright on the bottom surface of the receiving recess section 51.

The cap 40 and the holder 50 are fixed to each other by, for example, welding or bonding. When the cap 40 and the holder 50 are made of synthetic resin, the cap 40 and the holder 50 may be fixed by using, for example, heat welding. In the present example, it is described that the housing 20 is formed by two members, which are the cap 40 and the holder 50. However, instead of this, the housing 20 may be formed by one member or three members according to a manufacturing method to be employed. As a material of the valve element 30, it is preferable to use, for example, a rubber material and a thermoplastic elastomer.

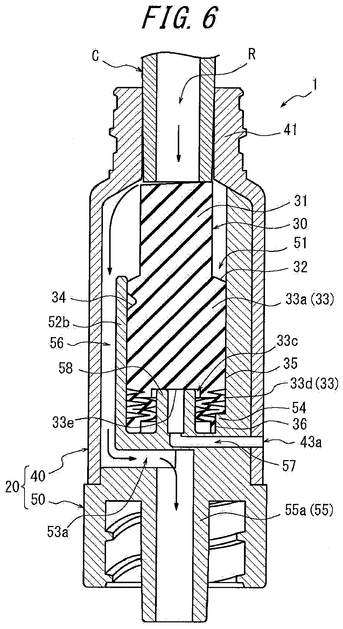

According to the configurations described above, as illustrated in Fig. 6, when a male connector C is connected to the male connector connection section 41, the head section 31 of the valve element 30 is pushed into the male connector connection section 41 by the male connector C while the head section 31 contracts the bellows-shaped circumferential wall 33d of the body section 33. In this case, it is possible to discharge air in the hollow section 33c of the body section 33 to the outside of the housing 20 through the holder side air passage 57 and the through hole 43a which are used as an air passage, so that it is possible to reduce force required to connect the male connector C to the male connector connection section 41 and make the connection easy.

When the bellows-shaped circumferential wall 33d is folded, the support protrusion 58 provided on the bottom surface of the receiving recess section 51 comes into contact with an end surface 33e, which faces the head section 31, of the hollow section 33c of the valve element 30, so that it is possible to prevent further deformation of the bellows-shaped circumferential wall 33d. When the head section 31 of the valve element 30 is further pushed into the male connector connection section 41 from this state, the notch 34 formed in the body section 33 of the valve element 30 is deformed so as to be crushed, so that the head section 31 of the valve element 30 is tilted.

As a result, it is possible to connect a fluid flow passage R in the male connector C to the fluid flow passage 55b in the medical instrument connection section 55 through the fluid connection passage 56 and the half-disk-shaped notch 53a which are formed outside the receiving recess section 51. At this time, the body section 33 of the valve element 30 is covered by the receiving recess section 51.

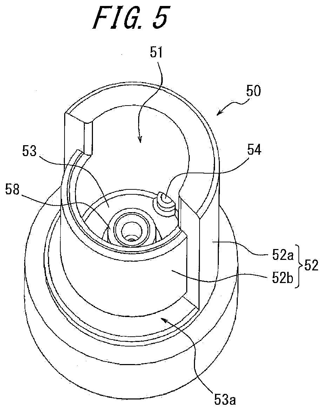

Therefore, as illustrated by arrows in Fig. 5, it is possible to suppress or prevent fluid flowing in the housing 20 from coming into contact with the body section 33 of the valve element 30, so that it is possible to suppress or prevent occurrence of contamination due to adhesion of fluid at the body section 33 of the valve element 30.

Further, in the present example, the seal protrusion 35 that is provided around the outer circumferential surface of the body section 33 of the valve element 30 is liquid-tightly and slidably attached to the inner circumferential surface of the receiving recess section 51 of the housing 20, so that it is possible to further suppress the fluid flowing in the housing 20 from invading the outer circumferential surface of the bellows-shaped circumferential wall 33d of the valve element 30. In this way, according to the present example, it is possible to suppress the invasion of the fluid to the bellows-shaped circumferential wall 33d where the fluid is easily attached and in particular there is a large risk of occurrence of contamination.

Further, a rotation preventing means (the protrusion 54 and the recess section 36) that prevents rotation of the body section 33 in a circumferential direction is provided between the body section 33 of the valve element 30 and the receiving recess section 51 of the housing 20, so that it is possible to consistently maintain a certain direction in which the head section 31 of the valve element 30 is tilted. Therefore, it is possible to consistently maintain a certain track in which the fluid in the medical connector 1 flows. Further, by the rotation preventing means, it is possible to position the notch 34 at the center of the second half circumferential part 52b in the circumferential direction, so that it is possible to guide the fluid from inside of the male connector C to the fluid connection passage 56 by a shortest track at all times . Therefore, it is possible to stably reduce flow resistance . As the rotation preventing means, it is possible to use welding or adhesion instead of the protrusion 54 and the recess section 36. In other words, the rotation of the body section 33 in the circumferential direction may be prevented by forming a welding or adhesion section between the body section 33 of the valve element 30 and the receiving recess section 51 of the housing 20.

Further, when the male connector C is removed along with release of connection of the male connector C from the male connector connection section 41, it is possible to expand and restore the bellows-shaped circumferential wall 33d of the body section 33 of the valve element 30. Therefore, it is possible to suppress or prevent the increase of volume of the fluid flow passage in the housing 20, which has been concerned to occur when the male connector C is removed, by expansion of the bellows-shaped circumferential wall 33d of the body section 33 of the valve element 30, so that it is possible to suppress or prevent occurrence of drawing-in of the fluid at the medical instrument connection section 55 when the male connector C is removed.

In the present example, the valve element 30 has the shoulder section 32 whose diameter increases in taper form from the lower end of the head section 31 to the upper end of the body section 33, so that even if the fluid pressure in the housing 20 increases when the medical instrument connection section 55 is connected to a tube body of a medical instrument (not illustrated in the drawings) and the male connector connection section 41 is in an unconnected state, it is possible to more reliably prevent the fluid in the housing 20 from leaking from the male connector connection section 41.

Further, in the present example, the diameter of the upper part 33a of the body section 33 of the valve element 30 is greater than the diameter of the head section 31, so that it is possible to reduce the volume of the fluid flow passage in the housing 20 by a volume corresponding to a difference between the diameters when the male connector C is removed. Therefore, by adjusting the difference between the diameters, it is possible to adjust the amount of fluid to be pushed out at the medical instrument connection section 55 when the male connector C is removed.

Here, if adjusting the difference so that a greater amount of fluid is pushed out, for example, when a drug is injected into a patient, there is a risk that an amount of the drug corresponding to the amount of fluid to be pushed out is injected into the patient. Therefore, it is preferable to adjust the amount of fluid to be pushed so that the amount of fluid to be pushed out is as small as possible. Specifically, the amount of fluid to be pushed out at the medical instrument connection section 55 when the male connector C is removed is preferable to be +0.05 ml (that is, 0.05 ml is pushed out) to 0 ml, and is more preferable to be adjusted to +0.01 ml (that is, 0.01 ml is pushed out) to 0 ml.

The description above only illustrates an embodiment of the present invention and various modifications can be added to claims. For example, in the above description, the body section 33 of the valve element 30 has the bellows-shaped circumferential wall 33d and the bellows-shaped circumferential wall 33d contracts when the male connector C is connected. However, it is not necessarily required to employ such a configuration, but any configuration can be employed as long as the body section 33 can contract. Further, in the example of the above description, the notch 34 is provided to the valve element 30. However, it is not necessarily required to employ such a configuration, but any configuration can be employed as long as the fluid flow passage R in the male connector C can be connected to the fluid flow passage 55b in the medical instrument connection section 55 through the fluid connection passage 56 when the male connector C is connected to the male connector connection section 41. In other words, when the notch 34 is not provided, for example, it is possible to employ a configuration in which the upper surface of the head section 31 of the valve element 30 has a sloped shape and a configuration in which a groove opened to the outer circumferential surface of the head section 31 is formed in the upper surface of the head section 31. When the notch 34 is provided to the valve element 30, the position where the notch 34 is provided is not limited as long as the fluid flow passage R in the male connector C can be connected to the fluid flow passage 55b in the medical instrument connection section 55 through the fluid connection passage 56 by tilting the head section 31 when the male connector C is connected. In other words, the notch 34 is not necessarily required to be provided near the upper end of the outer circumferential surface of the upper part 33a of the body section 33 as described above, but can be provided at any position above the seal protrusion 35. For example, the notch 34 can be provided in the outer circumferential surface of the head section 31.

### Reference Signs List

1 Medical connector
20 Housing
30 Valve element
31 Head section
32 Shoulder section
33 Body section
33a Upper part of the body section
33b Lower part of the body section
33c Hollow section of the body section
33d Bellows-shaped circumferential wall of the body section
33e End surface, which faces the head section, of the hollow section of the body section
34 Notch
35 Seal protrusion
36 Recess section (rotation preventing means)
40 Cap
41 Male connector connection section
42 Taper wall section
43 Outer circumferential wall section
43a Through hole (air passage)
50 Holder
51 Receiving recess section
52 Inner circumferential wall section
52a First half circumferential part
52b Second half circumferential part
53 Recess section bottom wall
53a Half-disk-shaped notch
54 Protrusion (rotation preventing means)
55 Medical instrument connection section
55a Cylindrical body
55b Fluid flow passage
56 Fluid connection passage
57 Holder side air passage (air passage)
58 Support protrusion
O Axis line
C Male connector
R Fluid flow passage in the male connector

## Claims

1. A medical connector (1) comprising:
a housing (20) including a male connector connection section (41) and a medical instrument connection section (55); and
a valve element (30) including a head section (31) that closes the male connector connection section (41) and a body section (33) that connects to the head section (31),
wherein the body section (33) of the valve element (30) is arranged in a receiving recess section (51) formed in the housing (20), and
by connecting a male connector (C) to the male connector connection section (41), the head section (31) of the valve element (30) is pushed in by the male connector (C) while the head section (31) contracts the body section (33), a fluid flow passage (R) in the male connector (C) is connected to a fluid flow passage (55b) in the medical instrument connection section (55) through a fluid connection passage (56) formed outside the receiving recess section (51), and the body section (33) is covered by the receiving recess section (51),
**characterized in that**
the body section (33) of the valve element (30) has a hollow section (33c) that opens at an end section opposite to the head section (31), and the body section (33) of the valve element (30) has a bellows-shaped circumferential wall (33d) that forms the hollow section (33c), and on a bottom surface of the receiving recess section (51) of the housing (20), a support protrusion (58) is formed which comes into contact with an end surface of the hollow section (33c) facing the head section (31) when the bellows-shaped circumferential wall (33d) is folded when the male connector (C) is connected and suppresses further deformation of the bellows-shaped circumferential wall (33d).

2. The medical connector (1) according to claim 1, wherein
on an outer circumferential surface of the valve element (30), a notch (34) is formed which connects the fluid flow passage (R) in the male connector (C) to the fluid flow passage (55d) in the medical instrument connection section (55) through the fluid connection passage (56) by tilting the head section (31) when the male connector (C) is connected, and
a rotation preventing means (36) that prevents rotation of the body section (33) in a circumferential direction is provided between the body section (33) of the valve element (30) and the receiving recess section (51) of the housing (20).

3. The medical connector (1) according to claim 1 or 2, wherein
the housing (20) has an air passage (57) that connects outside of the housing (20) with the hollow section (33c) of the valve element (30).

4. The medical connector (1) according to claim 3, wherein a seal protrusion (35) that is liquid-tightly and slidably attached to the receiving recess section (51) of the housing (20) is provided around an outer circumferential surface of the body section (33) of the valve element (30) at an end section of the bellows-shaped circumferential wall (33d) facing the head section (31).

## Patentansprüche

1. Medizinischer Verbinder (1), aufweisend:
ein Gehäuse (20), das einen Verbindungsabschnitt (41) für einen Steckverbinder und einen Verbindungsabschnitt (55) für ein medizinisches Instrument aufweist; und
ein Ventilelement (30), das einen Kopfabschnitt (31), welcher den Verbindungsabschnitt (41) des Steckverbinders und einen Körperabschnitt (33) umfasst, der mit dem Kopfabschnitt (31) verbunden ist,
wobei der Körperabschnitt (33) des Ventilelements (30) in einem Aufnahmeausnehmungsabschnitt (51) angeordnet ist, der in dem Gehäuse (20) ausgebildet ist, und
durch Verbinden eines Steckverbinders (C) mit dem Verbindungsabschnitt (41) des Steckverbinders, wobei der Kopfabschnitt (31) des Ventilelements (30) durch den Steckverbinder (C) eingeschoben wird, während der Kopfabschnitt (31) den Körperabschnitt (33) zusammenschiebt, wird ein Fluidströmungskanal (R) in dem Steckverbinder (C) mit einem Fluidströmungskanal (55b) in dem Verbindungsabschnitt (55) des medizinischen Instruments durch einen Fluidverbindungskanal (56), der außerhalb des Aufnahmeausnehmungsabschnitts (51) ausgebildet ist, verbunden und der Körperabschnitt (33) ist durch den Aufnahmeausnehmungsabschnitt (51) abgedeckt,
**dadurch gekennzeichnet, dass**
der Körperabschnitt (33) des Ventilelements (30) einen hohlen Abschnitt (33c) aufweist, der sich an einem dem Kopfabschnitt (31) gegenüberliegenden Endabschnitt öffnet, und der Körperabschnitt (33) des Ventilelements (30) eine balgförmige Umfangswand (33d) aufweist, die den hohlen Abschnitt (33c) bildet, und an einer Unterseite des Aufnahmeausnehmungsabschnitts (51) des Gehäuses (20) ist ein Abstützvorsprung (58) ausgebildet, der mit einer Stirnfläche des hohlen Abschnitts (33c), die dem Kopfabschnitt (31) zugewandt ist, in Kontakt kommt, wenn die balgförmige Umfangswand (33d) gefaltet ist, wenn der Steckverbinder (C) verbunden ist und eine weitere Verformung der balgförmigen Umfangswand (33d) unterbindet.

2. Medizinischer Verbinder (1) nach Anspruch 1, wobei
an einer äußeren Umfangsfläche des Ventilelements (30) eine Einkerbung (34) ausgebildet ist, die den Fluidströmungskanal (R) in dem Steckverbinder (C) mit dem Fluidströmungskanal (55d) in dem Verbindungsabschnitt (55) des medizinischen Instrumentes durch den Fluidverbindungskanal (56) verbindet, indem der Kopfabschnitt (31) geneigt wird, wenn der Steckverbinder (C) verbunden ist, und
ein Rotationsverhinderungsmittel (36), das eine Drehung des Körperabschnitts (33) in Umfangsrichtung verhindert, zwischen dem Körperabschnitt (33) des Ventilelements (30) und dem Aufnahmeausnehmungsabschnitt (51) des Gehäuses (20) vorgesehen ist.

3. Medizinischer Verbinder (1) nach Anspruch 1 oder 2, wobei
das Gehäuse (20) einen Luftkanal (57) aufweist, der außerhalb des Gehäuses (20) mit dem hohlen Abschnitt
(33c) des Ventilelements (30) verbunden ist.

4. Medizinischer Verbinder (1) nach Anspruch 3, wobei ein flüssigkeitsdicht und gleitend an dem Aufnahmeausnehmungsabschnitt (51) des Gehäuses (20) befestigter Dichtungsvorsprung (35) um eine äußere Umfangsfläche des Körperabschnitts (33) des Ventilelements (30) an einem Endabschnitt der balgförmigen Umfangswand (33d), der dem Kopfabschnitt (31) zugewandt ist, vorgesehen ist.

## Revendications

1. Raccord médical (1) comprenant :
un boîtier (20) comprenant une section de connexion de connecteur mâle (41) et une section de connexion d'instrument médical (55) ; et
un élément de soupape (30) comprenant une section de tête (31) qui ferme la section de connexion de connecteur mâle (41) et une section de corps (33) qui est reliée à la section de tête (31),
où la section de corps (33) de l'élément de soupape (30) est disposée dans une section d'évidement de réception (51) formée dans le boîtier (20), et
en reliant un connecteur mâle (C) à la section de connexion de connecteur mâle (41), la section de tête (31) de l'élément de soupape (30) est poussée par le connecteur mâle (C) pendant que la section de tête (31) contracte la section de corps (33), un passage d'écoulement de fluide (R) formé dans le connecteur mâle (C) est relié à un passage d'écoulement de fluide (55b) formé dans la section de connexion d'instrument médical (55) à travers un passage de connexion de fluide (56) formé à l'extérieur de la section d'évidement de réception (51), et la section de corps (33) est couverte par la section d'évidement de réception (51),
**caractérisé en ce que**
la section de corps (33) de l'élément de soupape (30) comprend une section creuse (33c) qui s'ouvre au niveau d'une section d'extrémité opposée à la section de tête (31), et la section de corps (33) de l'élément de soupape (30) comprend une paroi circonférentielle en forme de soufflet (33d) qui forme la section creuse (33c), et sur une surface inférieure de la section d'évidement de réception (51) du boîtier (20), est formée une saillie de support (58) qui vient en contact avec une surface d'extrémité de la section creuse (33c) tournée vers la section de tête (31) lorsque la paroi circonférentielle en forme de soufflet (33d) est repliée quand le connecteur mâle (C) est relié et supprime une déformation supplémentaire de la paroi circonférentielle en forme de soufflet (33d).

2. Raccord médical (1) selon la revendication 1, dans lequel
sur une surface circonférentielle extérieure de l'élément de soupape (30), est formée une encoche (34) qui relie le passage d'écoulement de fluide (R) du connecteur mâle (C) au passage d'écoulement de fluide (55d) de la section de connexion d'instrument médical (55) à travers le passage de connexion de fluide (56) en inclinant la section de tête (31) lorsque le connecteur mâle (C) est connecté, et
un moyen de prévention de rotation (36) qui empêche la rotation de la section de corps (33) dans une direction circonférentielle est prévu entre la section de corps (33) de l'élément de soupape (30) et la section d'évidement de réception (51) du boîtier (20).

3. Raccord médical (1) selon la revendication 1 ou 2, dans lequel
le boîtier (20) comprend un passage d'air (57) qui relie l'extérieur du boîtier (20) à la section creuse (33c) de l'élément de soupape (30).

4. Raccord médical (1) selon la revendication 3, dans lequel
une saillie d'étanchéité (35) qui est fixée de façon étanche au liquide et de façon coulissante à la section d'évidement de réception (51) du boîtier (20) est prévue autour d'une surface circonférentielle extérieure de la section de corps (33) de l'élément de soupape (30) au niveau d'une section d'extrémité de la paroi circonférentielle en forme de soufflet (33d) tournée vers la section de tête (31).
